# EUROPEAN PATENT APPLICATION

(11) **EP 4 098 752 A1**
(43) Date of publication of application: **07.12.2022**
(21) Application number: 22174167.1
(22) Date of filing: 18.05.2022
(51) Int. Cl.: C12Q 1/25

(54) **METHOD FOR OVERCOMING GENETIC RESISTANCE, ENHANCING THERAPEUTIC EFFICACY, AND MINIMIZING SAFETY RISKS ASSOCIATED WITH KINASE INHIBITOR THERAPY**

(30) Priority: 18.05.2021 US 202163190092 P
(71) Applicant: Nextcea Inc., Woburn, MA 01801 (US)
(72) Inventor: Hsieh, Frank, Woburn, 01801 (US)
(74) Representative: Purdylucey Intellectual Property

(57) **Abstract**

Described are methods for developing treatment plans for patients with cancer and other diseases based on drug binding interactions of kinase inhibitors. Also described are methods for determining the mutational status of a kinase and kinase occupancy.

## Description

### RELATED APPLICATION

This application claims priority to US Provisional Application No. 63/190,092, filed on May 18, 2021, the entire content of which is incorporated by reference herein.

### BACKGROUND

Given their important roles in cellular signaling, protein kinases are intensively pursued targets in pharmaceutical research, and a variety of small-molecule kinase inhibitors have been approved by the U.S. Food and Drug Administration (FDA) for clinical use. Although cancer has been the predominant indication for these drugs, focus within the pharmaceutical industry has expanded to include other illnesses, such as rheumatoid arthritis, autoimmune diseases, allergy, and Parkinson's disease.

Although many types of cancers are initially responsive to treatment, over time they can develop compensatory mechanisms that promote drug resistance. Resistance is a major problem hindering the clinical effectiveness of small-molecule kinase inhibitors. A drug's efficacy is influenced by its molecular target. Alterations of this target through genetic mutation, post-translational modification (PTM), or expression/activation level are among the ways cancer cells use to survive. For example, mutations can impede the effects of a kinase inhibitor by altering the contact points for its binding or perturbing the conformational state of the target enzyme. Such mutations disfavor inhibitor binding, and consequently limit efficacy. Genetic differences can exist at low (or non-detectable) levels within a cancer cell population before treatment and undergo positive selection during exposure to a clinically effective targeted agent. Small molecule kinase inhibitors are becoming increasingly used to treat cancer, particularly blood cancers (e.g., leukemia, lymphoma, and myeloma). New strategies are urgently needed to provide tailored personalized treatments for refractory or relapsed patients.

### SUMMARY

In one aspect, described herein is a method of determining target kinase occupancy of a test compound. The method includes: (1) obtaining a test sample, wherein the test sample is a biological sample from a test subject that has been administered the test compound, or is a biological sample that has been contacted with the test compound; (2) processing a first portion of the test sample to generate a population of a representative signature peptide that includes a bound signature peptide and a free signature peptide, wherein the bound signature peptide is a representative signature peptide bound to the test compound and the free signature peptide is the representative signature peptide free of the test compound, the representative signature peptide being a fragment of the target kinase that includes a binding site for the test compound; (3) determining in the processed sample the level of the bound signature peptide and the level of the free signature peptide; (4) obtaining the level of a bound target kinase and the level of a free target kinase in the test sample based on the level of the bound signature peptide and the level of the free signature peptide, respectively, the bound target kinase being the target kinase bound to the test compound and the free target kinase being the target kinase free of the test compound; and (5) obtaining the target kinase occupancy of the test compound, which is the level of the bound target kinase relative to the combined level of the level of the bound target kinase and the level of the free target kinase. The target kinase can be the intended target or an unintended target of the compound.

The method can further include, after step (1) but before step (2), contacting the test sample with a saturating amount of a reference compound that is capable of binding covalently and strongly to the target kinase at the binding site within the representative signature peptide, and determining in the processed sample the level of the representative signature peptide bound to the reference compound, wherein the test compound binds covalently to the target kinase. In some embodiments, the reference compound is the test compound that is stably isotope labeled or biotinylated.

In some embodiments, the method further includes obtaining a residual level of the target kinase in its active state but free from the test compound in the test sample based on the level of the representative signature peptide bound to the reference compound, wherein the level of the target kinase in its active state in the test sample is the combined level of the level of the bound target kinase and the residual level, and wherein the total level of the target kinase in the test sample is the combined level of the level of the target kinase in its active state and the level of the free target kinase; and obtaining the target kinase occupancy, which is the level of the bound target kinase relative to the total level of the target kinase. In some embodiments, the target kinase occupancy is a percent kinase occupancy calculated by 100 x (the level of the bound target kinase/the total level of the target kinase).

The method can further include, after step (1) but before step (2), contacting a second portion of the test sample with a saturating amount of a biotinylated probe that is capable of binding irreversibly to a site in the target kinase that includes the binding site of the test compound, processing the second portion of the test sample to generate a probe-bound signature peptide, which is the representative signature peptide bound to the biotinylated probe, and determining the level of the probe-bound signature peptide, wherein the test compound binds reversibly or non-covalently to the target kinase.

In some embodiments, the method further includes dissociating the test compound from the target kinase before contacting the second portion with the biotinylated probe.

In some embodiments, the method further includes obtaining the level of the target kinase in its active state based on the level of the probe-bound signature peptide, wherein the total level of the target kinase in the test sample is the combined level of the level of the target kinase in its active state and the level of the free target kinase; and obtaining the target kinase occupancy, which is the level of bound target kinase relative to the total level of the target kinase. In some embodiments, the target kinase occupancy is a percent kinase occupancy calculated by 100 x (the level of bound target kinase/the total level of the target kinase).

In some embodiments, the method further includes, after step (1), contacting a third portion of the test sample with a saturating amount of a reference compound that is capable of binding irreversibly to a site in the target kinase that includes the binding site of the test compound, and processing the third portion of the test sample to remove any protein not bound to the reference compound and excess reference compound; dissociating the test compound from any bound protein, precipitating the dissociated protein, and measuring the levels of the test compound and the reference compound in the supernatant, wherein the level of total protein bound to the test compound is given by the level of the test compound in the supernatant, and the amount of total protein not bound to the protein is given by the level of the reference compound in the supernatant, and the amount of total kinase equals the amount of the bound kinase plus the unbound kinase; and obtaining a total target occupancy, which is the level of bound total kinase relative to the level of total kinase. In some embodiments, the total target occupancy is a percent occupancy calculated by 100 x (the level of bound total kinase/the level of total kinase).

The method can further include obtaining an off-target occupancy of the test compound by subtracting the target kinase occupancy from the total target occupancy, wherein the off-target occupancy is the occupancy of the test compound to one or more unintended targets of the test compound.

In some embodiments, the method further includes, after step (1), fractionating out from a portion of the test sample a test compound-kinase complex by adding ammonium sulfate to precipitate out other proteins, recovering the supernatant, adding more ammonium sulfate to precipitate out the test compound-kinase protein complex, and collect the precipitated complex for analysis.

In some embodiments, the representative signature peptide is a peptide having up to 50 amino acids that contains MANGCLL (SEQ ID NO: 1), MANGSLL (SEQ ID NO: 2), MANGYLL (SEQ ID NO: 4), MANGRLL (SEQ ID NO: 5), MANGALL (SEQ ID NO: 6), MANGFLL (SEQ ID NO: 7), MANGTLL (SEQ ID NO: 8), MANGPLL (SEQ ID NO: 9), ISNGCLL (SEQ ID NO: 16), EFMEHGCLSDY (SEQ ID NO: 17), LPSGCL (SEQ ID NO: 18), LPSGCLRDF (SEQ ID NO: 19), MERGCLL (SEQ ID NO: 20), MENGCLL (SEQ ID NO: 21), and MPHGCLL (SEQ ID NO: 22).

In another aspect, described herein is a method of evaluating the likelihood of a subject responding to a candidate compound, the method comprising: (1) obtaining a test sample, wherein the test sample is a biological sample from the subject; (2) processing the test sample to generate a population of representative signature peptide that includes a modified signature peptide and a wild-type signature peptide, the representative signature peptide being a fragment of a kinase that is a target of the candidate compound, wherein the representative signature peptide includes a site that, if mutated or post-translationally modified, can affect responsiveness to the candidate compound, the modified signature peptide being the mutated or modified representative signature peptide, and the wild-type signature peptide being the representative signature peptide free from the mutation or modification; (3) determining in the processed sample the level of the modified signature peptide and the level of the wild-type signature peptide; (4) obtaining the presence or percentage of the mutation or modification based on the level of the modified signature peptide and the level of the wild-type signature peptide; and (5) evaluating the subject's likelihood of responding to the candidate compound based on the presence or percentage, wherein a presence or high percentage indicates a low likelihood. In some embodiments, the candidate compound is an inhibitor of a kinase.

In some embodiments, the mutation or modification is a BTK genetic mutation, optionally selected from C481S, C481F, C481Y, C481R, C481P, C481A, C481T, T474I, T474M, T474S, T474P, T316A, and L528W, or an auto-phosphorylation event selected from pY223, pY551, pY225, pY344, and pY361.

In one aspect, described herein is a method of evaluating the likelihood of a subject responding to a candidate compound, the method includes: (1) obtaining a test sample, wherein the test sample is a biological sample from the subject; (2) processing the test sample to generate a population of representative signature peptide that includes a phosphorylated signature peptide and a non-phosphorylated signature peptide, the representative signature peptide being (i) a fragment of a kinase that includes a portion of the activation segment containing the phosphorylatable residue that is phosphorylated upon activation of the kinase, or (ii) a fragment of a substrate including a phosphorylatable residue of the kinase, the phosphorylated signature peptide being the representative signature peptide phosphorylated at the phosphorylatable residue and the non-phosphorylated signature peptide is the representative signature peptide lacking the phosphorylation, wherein the kinase is a target of the candidate compound; (3) determining in the processed sample the level of the phosphorylated signature peptide and the level of the non-phosphorylated signature peptide; (4) obtaining the level of activated kinase or the level of phosphorylated substrate of the kinase based on the level of the phosphorylated signature peptide and the level of the non-phosphorylated signature peptide; and (5) evaluating the subject's likelihood of responding to the candidate compound based on the level or percentage of the activated kinase or the phosphorylated substrate of the kinase, wherein a higher level or percentage indicates that the subject has a likelihood of being resistant to the candidate compound.

In yet another aspect, a method of increasing the efficacy of a therapeutic compound that targets a kinase in a subject is described. The method includes: administering the compound to the subject under more than one condition; obtaining a target kinase occupancy of the compound in a biological sample obtained from the subject under each of the more than one condition using any of the methods described herein, wherein the target kinase is the intended target of the compound; and optimizing the dosing or regimen of the compound to achieve a desired target kinase occupancy in the subject based on the target kinase occupancy obtained under the more than one condition. In some embodiments, the subject is administered a combination therapy including more than one therapeutic compound that targets a kinase, and wherein the target kinase occupancy of each compound is determined, and dosing or regimen of the combination therapy is optimized based on the target kinase occupancy of each compound.

In one aspect, disclosed herein is a method of reducing a side effect of a therapeutic compound that targets a kinase in a subject, the method including: administering the compound to the subject under more than one condition; obtaining a target kinase occupancy of the compound in a biological sample obtained from the subject under each of the more than one condition using any of the methods described herein, wherein the target kinase is not the intended target of the compound; and optimizing the dosing or regimen of the compound to achieve minimal target kinase occupancy in the subject based on the target kinase occupancy obtained under the more than one condition.

In another aspect, described herein is a method of monitoring a subject being treated with a therapeutic compound that targets a kinase, the method including: obtaining a target kinase occupancy of the compound in a biological sample obtained from the subject after the compound is administered using any of the methods of described herein; and comparing the obtained target kinase occupancy with a corresponding pre-determined occupancy, the predetermined occupancy being obtained from a reference sample from a reference subject in which the compound exhibits a desired effect or from the same subject at a time point where the compound exhibits a desired effect; wherein the compound is determined to have the desired effect in the subject if the kinase target occupancy is equal to or falls above the pre-determined occupancy.

In another aspect, a method of monitoring in a subject an off-target effect of a therapeutic compound that targets a kinase is described. The method includes: obtaining a target kinase occupancy of the compound in a biological sample obtained from the subject after the compound is administered using any of the methods described herein, wherein the target kinase is not an intended target of the compound; and comparing the obtained target kinase occupancy with a corresponding pre-determined occupancy, the predetermined occupancy being obtained from a reference sample from a reference subject in which the compound causes an off-target effect or from the same subject at a time point where the compound causes an off-target effect; wherein the compound is determined to have the off-target effect in the subject if the kinase target occupancy equals to or falls above the pre-determined occupancy.

In any of the methods described herein, the target kinase occupancy can be determined in at least two samples obtained at different time points after treatment is administered to a subject.

The determined target kinase occupancy can be used to decide whether to continue to administer the compound, increase or decrease the dosage of the compound, modify the dosage regimen of the compound, stop administration of the compound, or administer an alternative treatment.

In any of the methods described herein, the test sample or biological sample can contain or be obtained from a bodily fluid (e.g., blood, plasma, serum, urine, or cerebrospinal fluid), a piece of tissue (e.g., brain, kidney, lung, or adipose tissue), cells (e.g., peripheral blood mononuclear cells (PBMCs), lymphocytes, leukocytes, nerve, fibroblasts, or other cell biopsies), induced pluripotent stem cells, or cell-derived vesicles. The sample can contain subject-derived cells such as peripheral blood cells (e.g., PBMCs, lymphocytes, leukocytes, lymphocytes, granulocytes, monocytes, and macrophages) or cancer cells (e.g., breast cancer cells, colorectal cancer cells, lung cancer cells, melanoma cells, pancreatic cancer cells, gallbladder cancer cells, and thyroid cancer cells), or a tumor cell line.

In any of the methods described herein, a signature peptide can be generated using any procedure that cleaves a protein into peptides, e.g., a specific proteolytic cleavage procedure (e.g., trypsin or chymotrypsin digestion), a non-specific proteolytic procedure (e.g., subtilisin, protanase, or ficin) or a chemical-based approach.

To determine the level of a signature peptide (e.g., a free peptide, peptide bound to a compound, a phosphorylated peptide, or a mutant or modified peptide) in any of the methods described herein, a ligand binding assay, liquid chromatographic (LC) based technique, capillary electrophoretic (CE) based technique, or a combined LC or CE mass spectrometric (MS) procedure (e.g., LC-MS, LC-MS/MS, CE-MS or CE-MS/MS) can be utilized.

In any of the methods described herein, the kinase can be selected from protein kinase B (PKB), tyrosine-protein kinase BLK, tyrosine-protein kinase BMX, Bruton's tyrosine kinase (BTK), epidermal growth factor receptor (EGFR), receptor tyrosine-protein kinase erbB-2 (ERBB2), receptor tyrosine-protein kinase erbB 4 (ERBB4), extracellular signal-regulated kinase (ERK), tyrosine-protein kinase Fgr (FGR), Fyn-related kinase (FRK), tyrosine-protein kinase Fyn (FYN), tyrosine-protein kinase HCK, tyrosine-protein kinase ITK/TSK/LYK, Janus kinase 3 (JAK3), lymphocyte-specific protein tyrosine kinase (LCK), tyrosine-protein kinase Lyn (LYN), ribosomal protein S6 kinase A3 (RPS6KA3), tyrosine-protein kinase Src (SRC), spleen tyrosine kinase (SYK), tyrosine-protein kinase Tec (TEC), tyrosine-protein kinase TXK, tyrosine-protein kinase Yes, and tyrosine-protein kinase ZAP-70.

In any of the method disclosed herein, the test compound or therapeutic compound can be one for treating a blood cancer (e.g., lymphoma, Hodgkin's lymphoma, Non-Hodgkin's lymphoma, Waldenstrom's macroglobulinemia (WM), mantle cell lymphoma, leukemia, childhood leukemia, acute lymphocytic leukemia (ALL), acute myelogenous leukemia (AML), chronic lymphocytic leukemia (CLL), chronic myelogenous leukemia (CML), myelodysplatic syndromes, and polycythemia vera), rheumatoid arthritis, autoimmune diseases, allergies, or Parkinson's disease. In some embodiments, the subject has a brain tumor (e.g. glioblastoma (GBM), neuroblastoma (NB), medulloblastoma (MB), and metastatic brain tumors) or another type of cancer (e.g., bladder cancer, breast cancer, cervical cancer, colon cancer, rectal cancer, kidney cancer, liver cancer, skin cancer, mesothelioma, lung cancer, oral cancer, ovarian cancer, pancreatic cancer, prostate cancer, sarcoma, thyroid cancer, non-hodgkin lymphoma, myeloma, or leukemia).

The details of one or more embodiments are set forth in the accompanying drawing and the description below. Other features, objects, and advantages of the embodiments will be apparent from the description and drawing, and from the claims.

### DESCRIPITION OF DRAWINGS

FIG. 1 is a schematic representation of an exemplary kinase target, Bruton's tyrosine kinase (BTK).
FIG. 2 is a scheme showing an exemplary signature peptide (SEQ ID NO: 1) of BTK bound to a non-covalent kinase inhibitor (ibrutinib).
FIG. 3 is a set of graphs showing concentrations of ibrutinib-bound and free BTK in the peripheral blood mononuclear cells (PBMCs) of cancer patients, as determined by LC-MS/MS analysis of signature peptides.
FIG. 4 is a graph showing the *in vitro* target occupancy of various small molecule covalent inhibitors of BTK, i.e., ibrutinib, acalabrutinib, spebrutinib, zanubrutinib, and tirabrutinib, in human leukocytes.
FIG. 5 is a graph showing BTK occupancy of zanubrutinib in a leukemia patient.
FIG. 6 is a graph showing BTK occupancy of acalabrutinib and its active metabolite ACP-5386 in various patients with leukemia.
FIG. 7 is a graph showing BTK occupancy in cancer patients on different doses of ibrutinib.
FIG. 8 is a graph showing BTK occupancy post dose at different time points.
FIG. 9 is a graph showing effect of doses on phosphorylation of Tyr551 in BTK.
FIG. 10 is a graph showing off-target binding of ibrutinib to tyrosine-protein kinase BMX BMX occupancy in leukemia patients.

### DETAILED DESCRIPTION

The disclosure is based, at least in part, on the unexpected discovery that the kinase occupancy of a covalent (i.e., irreversible) kinase inhibitor can be more accurately determined in an ex vivo assay by saturating a sample with an excess of a reference compound that is capable of binding covalently and strongly to a target kinase binding site. Another unexpected discovery was that the kinase occupancy of a non-covalent (i.e., reversible) kinase inhibitor could be more accurately determined by saturating a sample with a biotinylated probe or a reference compound capable of binding irreversibly to a target kinase binding site. The on and off-target occupancy of a kinase inhibitor can be determined by measuring signature peptides generated from bound and unbound kinases.

Levels of protein kinase signature peptides (free and drug-bound) can be used in a personalized approach to achieve positive clinical outcomes and reduce unintended side effects associated with small molecule kinase inhibitors in patients with cancer and other illnesses. The integrated approach takes into account a patient's intrinsic and acquired genetic differences. It allows physicians and researchers to more accurately predict the likelihood of a patient's response to a given treatment strategy and regimen. Although the following description and examples focus on kinase targeted cancer therapies, particularly the BTK inhibitor ibrutinib for the treatment of leukemia, similar approaches could be applied towards other conditions such as rheumatoid arthritis, autoimmune diseases, allergies, and Parkinson's disease.

The present disclosure describes a method to assess a patient's unique disease state and select an appropriate treatment option or regimen based on the status of the intended kinase target (i.e., its quantitative level, activation state, and the presence or percentage of genetic mutations), inhibitor engagement (% drug occupancy), and the inhibitor's selectivity. In this approach, the percentage of an enzyme occupied by a binding partner (reversible or irreversible) is determined based on the levels of representative signature peptides specific to the kinase of interest. These biomarkers contain the site(s) of engagement between the enzyme and the inhibitor and correspond to the enzyme in its active (bound and unbound) and inactive states. The methods described herein are useful for defining the relationship between occupancy and effect, optimizing dose administration in clinical settings, overcoming genetic resistance, enhancing therapeutic efficacy, and minimizing unwanted side effects in patients on kinase inhibitor therapies.

### Kinase Targeted Cancer Therapies

Protein kinases are enzymes that regulate the biological activity of other proteins by phosphorylation of specific amino acids with ATP as the source of phosphate. They play important roles in cell growth, division, metabolism, and apoptotic signaling pathways. Exemplary protein kinases include: protein kinase B (PKB, Akt), tyrosine-protein kinase BLK (B lymphocyte kinase), tyrosine-protein kinase BMX (bone marrow tyrosine kinase gene on chromosome X, also known as ETK), Bruton's tyrosine kinase (BTK), epidermal growth factor receptor (EGFR), receptor tyrosine-protein kinase erbB-2 (ERBB2, HER2), receptor tyrosine-protein kinase erbB-4 (ERBB4, HER4), extracellular signal-regulated kinase (ERK), tyrosine-protein kinase Fgr (FGR), Fyn-related kinase (FRK, tyrosine protein kinase 5), tyrosine-protein kinase Fyn (FYN), tyrosine-protein kinase HCK (hematopoietic cell kinase), tyrosine-protein kinase ITK/TSK/LYK (interleukin-2-inducible T-cell kinase), Janus kinase 3 (JAK3), lymphocyte-specific protein tyrosine kinase (LCK), tyrosine-protein kinase Lyn (LYN), ribosomal protein S6 kinase A3 (RPS6KA3), tyrosine-protein kinase Src (SRC), spleen tyrosine kinase (SYK), tyrosine-protein kinase Tec (TEC), tyrosine-protein kinase TXK (resting lymphocyte kinase, RLK), tyrosine-protein kinase Yes (YES1, Proto-oncogene c-Yes, p61-Yes), and tyrosine-protein kinase ZAP-70 (ZAP70, S-locus receptor kinase, SRK).

Dysregulated kinase activity is implicated in the carcinogenesis and metastases of various types of cancer, wherein kinases regulate cancer cell growth and help them to survive. These features make kinases (e.g., Bruton's protein-tyrosine kinase, BTK) attractive targets in treating cancer, particularly the blood cancers (e.g., leukemia, lymphoma, and myeloma). Conditions or disorders that may be treated by kinase inhibitors include: blood cancers such as lymphoma, Hodgkin's lymphoma, Non-Hodgkin's lymphoma, Waldenstrom's macroglobulinemia (WM), mantle cell lymphoma, leukemia, childhood leukemia, acute lymphocytic leukemia (ALL), acute myelogenous leukemia (AML), chronic lymphocytic leukemia (CLL), chronic myelogenous leukemia (CML), multiple myeloma, myelodysplatic syndromes, and polycythemia vera; brain cancers such as glioblastoma, neuroblastoma, medulloblastoma, and metastatic brain tumors; other types of cancers such as lung cancer, breast cancer, gastro-oesphageal cancer, pancreatic cancer, ovarian cancer, prostate cancer, bladder cancer, renal cell carcinoma, cervical cancer, colon cancer, rectal cancer, liver cancer, skin cancer, mesothelioma, sarcoma, and thyroid cancer; and other illnesses such as rheumatoid arthritis, autoimmune diseases, allergy, and Parkinson's disease.

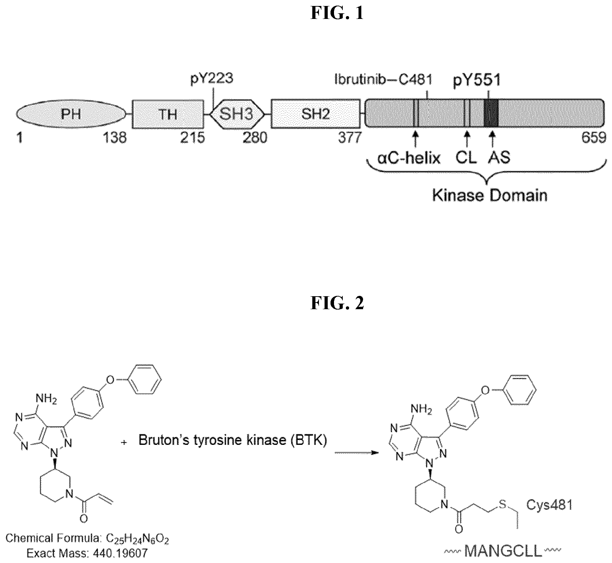

Kinases contain an active site to bind and orient substrate proteins for phosphorylation, a binding site for Mg2+-ATP (phosphate donor), as well as various allosteric (i.e., regulatory) sites. See, Avendaño C, Menéndez JC. Medicinal Chemistry of Anticancer Drugs, 1st Edition 2008; and Litalien et al., Pediatric Critical Care (Fourth Edition), 2011, Chapter 117, Pages 1553-1568. Also see Fig. 1. For most kinases, phosphorylation of a residue within the active segment (e.g., Tyr551 for BTK) converts the inactive (i.e., compacted) enzyme to an active one. Proper positioning of the peptide/protein substrate and ATP within the active site, usually in an exposed groove or pocket of the enzyme, enables the catalytic reaction to occur. The status of the activation segment exposed/compacted (i.e., active/inactive) is often used to represent the major conformational state of the enzyme.

Given their role in cancer biology, substantial efforts have been directed at blocking the function of protein kinases, and a variety of small-molecule kinase inhibitors have been approved by the U.S. Food and Drug Administration (FDA) for clinical use. Exemplary kinase inhibitors include: Abemaciclib, Acalabrutinib, Afatinib, Alectinib, Axitinib, Baricitinib, Binimetinib, Bosutinib, Brigatinib, Cabozantinib, Ceritinib, Cobimetinib, Crizotinib, Dabrafenib, Dacomitinib, Dasatinib, Encorafenib, Entrectinib, Erdafitinib, Erlotinib, Everolimus, Fostamatinib, Gefitinib, Gilteritinib, GNE-431, Ibrutinib, Lapatinib, Larotrectinib, Lenvatinib, Lorlatinib, Midostaurin, Neratinib, Netarsudil, Nilotinib, Nintedanib, Osimertinib, Palbociclib, Pazopanib, Pexidartinib, Ponatinib, Regorafenib, Ribociclib, Ruxolitinib, Sirolimus, Sorafenib, Spebrutinib, Sunitinib, Temsirolimus, Tirabrutinib, Tofacitinib, Trametinib, Vandetanib, Vecabrutinib, Vemurafenib, and Zanubrutinib. These inhibitors target kinome members such as EGFR, ERBB2, ERBB4, BLK, BTK, BMX, ITK, JAK3, TEC, TXK, VEGFRs, Kit, PDGFRs, ABL, SRC, and mTOR. Most small-molecule kinase inhibitors in clinical use target the ATP-binding pocket (types I and II) of an enzyme. A few of the non-ATP competitive inhibitors target allosteric (i.e., regulatory) sites (type III), or other pockets on an enzyme (type IV). The different types of inhibitors (Type I-IV) can be covalently or non-covalently bound.

### Assays for Determining Therapeutic Resistance

Most types of kinase inhibitors are subject to genetic mutation resistance, rendering some patients less responsive. Evidence suggests that these mutations can exist at low levels before drug treatment and undergo positive selection during exposure to a clinically effective targeted agent. In the case of ibrutinib, mutation of *BTK* itself seems to be an important molecular mechanism of underlying disease progression while on therapy, with a high frequency in resistant patients. See, Woyach et al., N Engl J Med. 2014 Jun 12;370(24):2286-94; Lampson and Brown, Expert Rev Hematol. 2018 March; 11(3):185-194; and Sharma et al., Oncotarget, 2016 7(42):68833-68841.

Ibrutinib occupies the ATP-binding pocket of BTK when the enzyme assumes its active conformation. Nearly all of the reported BTK mutations associated with ibrutinib resistance affect the Cys481 hotspot, the residue to which ibrutinib covalently binds (e.g., Cys481Ser/Tyr/ Arg/Ala/Phe/Thr). Rare mutations at other BTK sites, including the Thr474 gatekeeper which ibrutinib forms a hydrogen bond with (e.g., Thr474Phe/ Ile/Ser, Thr316Ala, and Leu528Trp) have also been detected in relapsed/refractory patients on ibrutinib therapy. See, Sharma et al., Oncotarget, 2016 7(42):68833-68841; and Hamasy et al., Leukemia. 2017 Jan;31(1):177-185.

In addition to altering the contact points for drug binding, genetic mutations can alter the conformational state of a kinase drug target, rendering it more or less active. Typically, these mutations either promote adoption of an active conformation by the kinase (i.e., upregulate kinase activity) or prevent conformational changes required for drug binding.

Although genetic sequencing analyses have been used routinely and reliably to screen for mutations of *BTK,* they do not always accurately predict patients' responses. Mutations of *BTK* are also frequent among those with sustainable response. With irreversible inhibitors, like ibrutinib, even mutants that react more slowly with the inhibitor may become fully inactivated given long enough contact with the drug. For example, a complicating factor of the BTK Cys481Ser mutation is the conversion of ibrutinib from an irreversible to a reversible (yet still functional) inhibitor. See, Lampson and Brown, Expert Rev Hematol. 2018 March; 11(3):185-194. Additional complicating factors associated with drug resistance are compensatory amplification and oncogenic activation of the target by cancer cells.

A method described herein can be used to develop a personalized treatment plan based on the presence and levels of target mutations in a patient's peripheral blood cells prior to, during, and after therapy. The method includes (1) identifying representative signature peptides that scan the mutation (or PTM) of interest, (2) processing a test sample from a candidate subject or patient to generate the signature peptides, (3) determining the presence or percentage of the mutation (PTM) based on the signature peptide levels in the processed sample, (4) evaluating the subject's likelihood of responding to a treatment, and (5) administering an appropriate therapy (or discontinuing an inappropriate treatment). For example, acquired resistance can develop gradually where subpopulations of cells acquire or already have the mutations enabling them to emerge under selective drug pressure. The appearance of or an increase in percentage of a mutated peptide, e.g., MANGSLL (SEQ ID NO: 2), over a wild-type peptide, e.g., MANGCLL (SEQ ID NO: 1), would indicate that a patient has acquired resistance to ibrutinib, presenting the physician with an opportunity to select an alternate therapy.
BTK amino acid sequence (SEQ ID NO: 3)

In this aspect, a signature peptide is a fragment of a kinase containing 5 to 50 (e.g., 5, 10, 15, 20, 25, 30, 35, 40, 45, 50, 5 to 10, 10 to 15, 15 to 20, 20 to 25, or 25 to 30) amino acids that includes a residue that, if mutated (e.g., by substitution or deletion) or modified (e.g., by post-translational modification), can affect responsiveness to an inhibitor of the kinase. A signature peptide can contain a wild-type residue or a mutated residue. Exemplary signature peptides include peptides containing a wild-type residue and peptides containing mutated counterparts, e.g., MANGCLL (SEQ ID NO: 1), MANGSLL (SEQ ID NO: 2), MANGYLL (SEQ ID NO: 4), MANGRLL (SEQ ID NO: 5), MANGALL (SEQ ID NO: 6), MANGFLL (SEQ ID NO: 7), MANGTLL (SEQ ID NO: 8), MANGPLL (SEQ ID NO: 9), YTVSVFAK (SEQ ID NO: 10), YAVSVFAK (SEQ ID NO: 11), NCLVNDQGVVK (SEQ ID NO: 12) and NCWVNDQGVVK (SEQ ID NO: 13).

In any of the methods described herein, a signature peptide can be generated using any procedure that cleaves a protein into peptides, e.g., a specific proteolytic cleavage procedure (e.g., trypsin or chymotrypsin digestion), a non-specific proteolytic procedure (e.g. subtilisin, protanase, or ficin) or a chemical-based approach. The level of a signature peptide can be quantitated using methods such as ligand-binding assay, liquid chromatographic (LC), capillary electrophoretic (CE) based technique, or a combined LC or CE mass spectrometric (MS) procedure (e.g., LC-MS, LC MS/MS, CE-MS or CE-MS/MS). The measured peptide concentration can be converted to protein concentration and normalized by total protein.

### Kinase Target Activation or Activity

Protein kinases contain hydrophobic catalytic and regulatory spines and collateral shell residues that are required to assemble the active enzyme. There are two general kinds of conformational changes associated with most protein kinases. The first conformational change involves the formation of the intact regulatory spine to form an active kinase. The regulatory spine will properly position the peptide or protein substrate, and the catalytic spine will position ATP within the active site thus enabling catalytic activity. At this stage, the enzyme exists in a stabilized compacted conformation, i.e., it is auto-inhibited.

The second conformational change occurs in active kinases as they toggle between open and closed (i.e., compacted) conformations during their catalytic cycles. For example, PIP3-mediated dimerization of BTK at the cell membrane disrupts the compact formation of BTK and leads to the trans-phosphorylation of Tyr551 within the active segment of one or both proteins thereby leading to an activated state. The phosphorylation of Tyr223 also occurs; this modification does not alter the catalytic activity of BTK, but it may change the selectivity towards it binding partners. As described herein, activation segment exposed/compacted (active/inactive) are used to represent the major conformational states of the enzyme.

BTK is overexpressed in B-cell malignancies. It plays an important role in the development, activation, signaling, proliferation and survival of B-lymphocytes and is a well-known downstream mediator of the B cell receptor (BCR). Activated BTK participates in the B cell antigen receptor signaling pathway (i.e., BTK directly phosphorylates and activates phospholipase Cγ2 (PLCγ2) at residues Tyr753 and Tyr759), which leads to the activation of the Ras/RAF/MEK/ERK signaling module (promoting cell growth and proliferation) and the nuclear factor NF-κB pathway (immune response). Additionally, BTK plays an essential role in the chemokine-mediated homing and adhesion of B cells. Besides its role in BCR signaling, BTK participates in chemokine and Toll-like receptor signal transduction. Fcγ receptors (FcγR) on immune cells (e.g., macrophages) play an important role in tumor-specific antibody-mediated immune responses and many such responses involve BTK.

Described herein is a method for determining the activation status and activity of a target kinase in a patient sample. The method can include (1) identifying signature peptides that scan the activation segment of a target in its active or inactive state, e.g., YVLDDEY[Pho]TSSVGSK (SEQ ID NO: 14) and YVLDDEYTSSVGSK (SEQ ID NO: 14) of BTK, and/or the phosphorylation site of the kinase substrate, e.g., For BTK, DINSLYDVSR (SEQ ID NO: 15) and DINSLY[Pho]DVSR (SEQ ID NO: 15) of PLCγ2), (2) processing a test sample from a candidate subject or patient to generate the signature peptides, (3) determining the presence or percentage of the activated enzyme and/or phosphorylated substrate based on the signature peptide levels in the processed sample. The results can be used to evaluate the subject's likelihood of responding to a treatment, and to determine an appropriate therapy or to decide to discontinuing an inappropriate treatment. For example, an increase in a kinase (e.g., BTK) activation and activity level could represent sustained signaling through the signaling (e.g., BCR) pathway even in the presence of a kinase inhibitor (e.g., ibrutinib). An increase in the percentage of phosphorylated signature peptides (e.g., BTK or PLCγ2 peptides) over non-phosphorylated signature peptides would indicate a patient's resistance to a kinase inhibitor, presenting an opportunity to change the dose or select an alternate therapy.

In this aspect, a signature peptide is a fragment of a kinase containing 5 to 50 (e.g., 5, 10, 15, 20, 25, 30, 35, 40, 45, 50, 5 to 10, 10 to 15, 15 to 20, 20 to 25, or 25 to 30) amino acids that includes a portion of the activation segment containing the residue that is phosphorylated upon activation of the kinase. Alternatively, the signature peptide can be a fragment of a kinase substrate containing 5 to 50 amino acids that includes a target phosphorylatable residue of the kinase. A signature peptide can be phosphorylated or non-phosphorylated.

### Drug Occupancy and Selectivity

In another aspect, the present disclosure describes methods which use representative signature peptides derived from patient samples as biomarkers to determine drug occupancy and selectivity. The signature peptides are used to measure the concentrations of a kinase in its active (drug-bound or free) and inactive states. The selected peptides are specific to the drug binding site(s) of the kinase of interest. Different inhibitors can bind covalently and/or non-covalently at different sites of the same kinase. For example, ibrutinib makes hydrogen bonds with BTK at the gatekeeper Thr474 as well as the first (E475) and third (M477) hinge residues, and covalently binds with nearby Cys481 to form an inactive adduct. Acalabrutinib covalently binds Cys481 in BTK as well, but with reduced reactive intrinsic reactivity compared to ibrutinib. Vecabrutinib is a non-covalent BTK inhibitor that reversibly binds to the gatekeeper Thr474. It was unexpectedly discovered that the % drug occupancy of a kinase inhibitor can be more accurately determined by saturating the patient sample with an excess of a strong irreversible binding partner (e.g., ibrutinib-d5 for BTK or biotinylated probe), thereby binding any residual active kinase in the sample.

Target occupancy can also be correlated with the mutational status or activation status of a kinase as described above. For example, drug occupancy and mutational status and/or activation status can be determined in a sample or samples from the same subject.

In any of the methods described herein, a signature peptide can be generated using a procedure that cleaves a protein into peptides, e.g., a specific proteolytic cleavage procedure (e.g., trypsin or chymotrypsin digestion), or a non-specific proteolytic procedure (e.g., subtilisin, protanase, or ficin) or chemical-based approach. The level of a signature peptide or protein can be quantitated using methods such as a ligand-binding assay, liquid chromatographic (LC), capillary electrophoretic (CE) based technique, or a combined LC or CE mass spectrometric (MS) procedure (e.g., LC-MS, LC MS/MS, CE-MS or CE-MS/MS). A measured peptide concentration can be converted to protein concentration and normalized by total protein.

### I. Covalent protein kinase inhibitors

Covalent (irreversible) small molecule kinase inhibitors (e.g., ibrutinib, acalabrutinib, zanubrutinib, tirabrutinib, and evobrutinib) are typically designed by integrating a reaction moiety (warhead) and a reversible part, which non-covalently anchors to the kinase target (e.g., the ATP-binding site). The warhead improves the binding affinity and selectivity by forming a covalent interaction with a kinase reactive residue. Most frequently, the covalent reactive residues are cysteines located near the ATP binding site (e.g. Cys481 for BTK, Cys319 for BLK, Cys496 for BMX, Cys449 for TEC, and Cys350 for TXK).

The active kinase in a patient sample can be captured by adding an excess of a strong irreversible binding partner (e.g., ibrutinib-d5 or -biotin conjugate). For example, all of the active kinase molecules in a sample from a drug-naive patient will bind to the biotin-linker probe and, due to the strong affinity between biotin and streptavidin, can be purified from the sample and quantified. Alternatively, within the sample, an active enzyme can be distinguished from an inactive enzyme by its binding affinity for the strong isotopic labelled-probe. In a patient on a covalent kinase inhibitor therapy, the strong binding probe will bind any residual active kinase in the sample.

Described herein is a method of determining the occupancy of a test compound in a human subject administered with the test compound or a test sample that has been contacted with the rest compound. The method includes the steps of (1) obtaining the test sample, (2) processing the test sample to generate a signature peptide specific to the drug binding site of interest, and (3) determining the level and status of the signature peptide (bound or free) in the processed sample, wherein the occupancy of the test compound is determined based on the level and status of the signature peptides relative to its total value.

Here, a signature peptide is a fragment of a kinase containing 5 to 50 (e.g., 5, 10, 15, 20, 25, 30, 35, 40, 45, 50, 5 to 10, 10 to 15, 15 to 20, 20 to 25, or 25 to 30) amino acids that includes a residue which a kinase inhibitor targeting that kinase can bind to covalently. A signature peptide may be free or bound to a kinase inhibitor. In any of the methods described herein, a signature peptide can be generated using any procedure that cleaves a protein into peptides, e.g., a specific proteolytic cleavage procedure (e.g., trypsin or chymotrypsin digestion), or a non-specific proteolytic procedure (e.g. subtilisin, protanase, or ficin) or chemical-based approach. Exemplary signature peptides for BTK include peptides containing a wild-type residue and peptides containing mutated counterparts, e.g., MANGCLL (SEQ ID NO: 1), MANGSLL (SEQ ID NO: 2), MANGYLL (SEQ ID NO: 4), MANGRLL (SEQ ID NO: 5), MANGALL (SEQ ID NO: 6), MANGFLL (SEQ ID NO: 7), MANGTLL (SEQ ID NO: 8), and MANGPLL (SEQ ID NO: 9). Additional exemplary signature peptides include: ISNGCLL (SEQ ID NO: 16) for BMX, EFMEHGCLSDY (SEQ ID NO: 17) for ITK, LPSGCL (SEQ ID NO: 18) for JAK3-1, LPSGCLRDF (SEQ ID NO: 19) for JAK3-2, MERGCLL (SEQ ID NO: 20) for TEC, MENGCLL (SEQ ID NO: 21) for TXK, and MPHGCLL (SEQ ID NO: 22) for ERBB4. A signature peptide can be designed for any target kinase if the binding residue(s) between the kinase and a kinase inhibitor are known.

In the absence of a kinase inhibitor (i.e., binding partner), 100% of the kinase in the test sample is unbound (i.e., free). When the test sample is treated with a saturating level of a strong kinase binding binder, e.g., ibrutinib-d5 (an analog of the BTK inhibitor ibrutinib), all of the active kinase in the sample become bound to ibrutinib-d5. The concentration of the active kinase is measured based on the level of a representative signature peptide, e.g., MANGC^{[Ibrutinib-d5]}LL (SEQ ID NO: 1) for BTK. The level of any inactive kinase is measured by the corresponding free peptide, e.g., MANGCLL (SEQ ID NO: 1).

In the presence of a covalent irreversible inhibitor (e.g., ibrutinib and acalabrutinib), some or all of the kinase in the test sample can be already inhibitor-bound. When the sample is treated with a saturating level of a strong binding partner, any residual active kinase will bind to the strong binding partner. The levels of the kinase signature peptides in different states (i.e., free, bound to the inhibitor, or bound to the strong binding partner), e.g., MANGC^{[Ibrutinib]}LL (SEQ ID NO: 1), MANGC^{[Ibrutinib-d5]}LL (SEQ ID NO: 1), and MANGCLL (SEQ ID NO: 1) for BTK, are measured to determine the concentrations of the kinase in its active (bound and free) and inactive states. The free peptide represents the inactive state, the peptide already bound to the inhibitor represents the active and bound state, and the peptide bound to the strong binding partner represents the active and free state.

A measured peptide concentration can be converted to protein concentration and normalized by total protein.

The percent kinase occupancy of the test compound is given by %Occupancy = 100 x (bound amount/total amount); and full kinase occupancy is confirmed at 100%. The total amount of kinase in the processed sample is given by [total amount = active kinase amount (bound and unbound states) + inactive kinase amount].

### II. Reversible (non-covalent) kinase inhibitors

Also described herein is a method for determining the occupancy of non-covalent (i.e., reversible) kinase inhibitors (e.g., vecabrutinib, Loxo-305, ARQ-531). The method includes obtaining a test sample from a human subject that has been administered a test compound or a sample that has been contacted with a test sample, processing the sample to obtain signature peptides, and determining the levels of the signature peptides, wherein the occupancy of the test compound is determined based on the level and status of the signature peptide representing the kinase in its bound state relative to its total value.

Here, a signature peptide is a fragment of a kinase containing 5 to 50 (e.g., 5, 10, 15, 20, 25, 30, 35, 40, 45, 50, 5 to 10, 10 to 15, 15 to 20, 20 to 25, or 25 to 30) amino acids that includes a residue which a kinase inhibitor targeting that kinase can bind to non-covalently. A signature peptide may be free or bound to a kinase inhibitor. A signature peptide can be designed for any target kinase if the binding residue(s) between the kinase and a kinase inhibitor are known.

In the presence of a non-covalent reversible inhibitor, some or all of the kinase molecules in the test sample can be inhibitor-bound. Signature peptides generated from the sample or a portion of the sample are measured to determine the concentrations of the kinase in its active bound state (e.g., a peptide containing the Thr474^{[vecabrutinib]} gatekeeper residue for BTK reversibly bound to vecabrutinib). The level of the corresponding free peptide containing the unbound residue (e.g. Thr474) is used to determine the amount of unbound active and inactive kinase.

In a separate experiment using the same sample (e.g., a portion of the sample), the inhibitor can be dissociated from the kinase binding site (i.e., the binding pocket is opened), and the sample is contacted with an excess of a biotinylated-linker probe. Signature peptides can then be generated. The total amount of active kinase is given by the level of the signature peptide bound to the biotinylated-probe (i.e., containing the Thr474^{[biotinylated probe]} residue). The amount of active kinase (unbound state) = total active kinase - active kinase (bound state). A measured peptide concentration can be converted to protein concentration and normalized by total protein.

In one embodiment, non-covalent drug-protein complexes can be fractionated out of a test sample or a portion of the test sample by adding a specific amount of ammonium sulfate to precipitate out non-desirable proteins. The supernatant can be recovered, and then more ammonium sulfate can be added to the supernatant to precipitate out the desired drug-protein complex. The precipitate can be isolated by centrifugation for further analysis.

In another aspect, the method can be used to profile the non-covalent interactions of a kinase inhibitor with a specific kinase or different kinases based on the profile of multiple signature peptides (e.g., each including one or more potential kinase binding sites) analyzed as a screening panel.

### III. Kinase Inhibitor Selectivity (off-target occupancy)

Kinase inhibitors can be active against multiple kinases and have diverse selectivity profiles. For example, regorafenib is known to target at least 14 kinases, whereas lapatinib, visodegib, axitinib, and ruxolitinib have more limited spectrums of kinase inhibition. Advantages to the design of potent inhibitors with desired selectivity profile include improvements in tolerability and therapeutic index, enabling the development of combination regimens, validation of new targets and molecular dependencies of tumors, and assessment of mechanisms of action.

Described herein is a method for determining the off-target occupancy of a kinase inhibitor. In one aspect, the method includes obtaining a test sample from a test subject that has been administered a test compound or a test sample that has been contacted with a test compound, processing the sample to generate a signature peptides specific to the drug binding sites of potential off-targets, measuring the levels and status, e.g., active (bound and unbound) and inactive, of signature peptides representative of off-target kinases in the test sample, and determining off-target occupancy (covalent or non-covalent) as described above.

In another aspect, described herein is a method of determining the off-target occupancy of a non-covalent kinase inhibitor (the test compound) by comparing its target and total occupancies in a sample. The off-target % occupancy = total %occupancy - target %occupancy. In this method, the target occupancy of the test compound is determined by a signature peptide-based approach as described above. Total %occupancy is determined in a separate experiment using the same sample or a portion of the sample as follows. The sample or portion of sample is spiked with an excess of an analog of the test compound (e.g., vecabrutinib-d3 for vecabrutinib) or another reversible binder targeting the same binding site. The sample is processed and then any unbound protein and excess analog are removed (e.g., washed) from the sample. A solvent is then added to dissociate the non-covalent complexes and precipitate the protein. The concentrations of the test compound and its analog (or other non-covalent binder) are measured in the supernatant. The total %occupancy of the test compound is given by %occupancy = 100 x (bound protein amount/total protein amount); and full occupancy is confirmed at 100%. The amount of bound protein is given by the level of the test compound (e.g., vecabrutinib). The amount of unbound protein is given by the level of the spiked analog (e.g., vecabrutinib-3), and total protein amount equals the bound protein amount plus the unbound protein amount.

In other words, the same methods described above for determining drug occupancy for covalent protein kinase inhibitors and reversible protein kinase inhibitors can be used to assess off-target occupancy using signature peptides from off-target kinases.

The level of a protein, test compound or analog can be quantitated using methods such as a ligand-binding assay, liquid chromatographic (LC), capillary electrophoretic (CE) based technique, or a combined LC or CE mass spectrometric (MS) procedure (e.g., LC-MS, LC MS/MS, CE-MS or CE-MS/MS).

### Methods of Enhancing Drug Efficacy

Also described herein are methods to enhance drug efficacy which utilize signature peptides of certain protein kinases as biomarkers to determine the occupancy and selectivity of small-molecule kinase inhibitors. The methods can be used in research and clinical settings for (1) defining the relationship between occupancy and effect, (2) increasing the efficacy of kinase-targeted therapies, (3) managing and monitoring patient treatment, and (4) evaluating combination therapies.

### I. Defining the relationship between occupancy and effect

The translation of target occupancy to drug pharmacodynamics depends on the relationship between occupancy and effect, which in turn depends on target vulnerability. Target vulnerability refers to what fraction of the intended kinase target has to be engaged to elicit the desired clinical outcome. One goal is to achieve a desired target kinase occupancy (i.e., the degree of engagement that is required to generate the desired response). For a low vulnerability target, a full or desired physiological effect of the drug may require close to 100% target engagement (e.g., 90% to 95%), whereas less engagement (e.g., 50% or less) may be sufficient for a high vulnerability target. See, Tonge, ACS Chemical Neuroscience 2018 9:29-39. In one aspect, the methods described herein can be used to evaluate the relationship between kinase occupancy and effect and determine appropriate dose levels for patients. For example, kinase occupancy can be determined in a subject administered a kinase inhibitor under different conditions (e.g., frequency, dosages, dosage regimens) to assess the relationship between kinase occupancy and effect. An optimal dosage or regimen can be selected to achieve a desired target engagement (e.g., at least 30%, 35%, 40%, 45%, 50%, 55%, 60%, 65%, 75%. 80%, 85%, 90%, or 95%).

### II. Increasing the efficacy of a kinase-targeted therapy

Target occupancy (binding) and selectivity are key aspects for optimizing drug dosing level and frequency. In one aspect, described herein is a method that includes (1) administering a kinase inhibitor under more than one condition (e.g. different dose levels, frequencies, and/or durations) to a test subject or subjects, (2) determining kinase target occupancy in sample(s) obtained from the test subject(s) based on signature peptide levels as described herein, (3) correlate target occupancy with clinical outcome (e.g., white blood cell count), and (4) optimizing the dosing conditions or regimens based on the results in order to increase efficacy.

### III. Managing and monitoring patient treatment

In another aspect, described herein is a method of monitoring a patient undergoing a kinase-targeted therapy. The method includes the steps of (1) determining the kinase target occupancy of a compound in a sample obtained from the patient (based on signature peptide levels as described herein) after the therapy is administered, and (2) comparing the determined level with a corresponding pre-determined level, the predetermined level being obtained from a reference sample wherein the compound is expected to have the desired outcome in the patient if the kinase target occupancy falls above the pre-determined level. The reference level can be obtained from a control subject having the desired outcome or the same patient at a time point exhibiting the desired outcome.

### IV. Assessing combination therapies

Also described herein is a method for exploring drug combinations on the same target kinase (e.g., different combinations of covalent and/or non-covalent compounds bound to the same or different sites of the same target) or different targets within a single pathway or parallel kinase pathways. The method includes the steps of (1) determining the target occupancies of multiple compounds in a sample obtained from the patient after a combination therapy is administered using the methods described herein, and (2) optimizing the dosing regimens based on the occupancy levels, and optionally, clinical outcome to increase efficacy.

### Minimizing Off-Target Side effects

Although small molecule kinase inhibitors have demonstrated meaningful benefits in clinical trials, there are still questions about their side effects over time. For example, the BTK inhibitor ibrutinib produced marked efficacy in chronic lymphocytic leukemia (CLL) clinical trials and is approved by the US FDA for the therapy of any CLL patient in any line of therapy. However, it is not selective for BTK and is associated with well-recognized toxicities, e.g., atrial fibrillation, infection, pneumonitis, bleeding, and arthralgia. Managing toxicities to keep patients on the drug long enough to achieve a deep response is critical for ibrutinib benefit, and at present, little is known about the potential durability of response after ibrutinib discontinuation for adverse events. See, Brown, Blood 2018 Jan 25; 131(4):379-386

Described herein are methods to minimize the side effects of kinase inhibitors. The methods utilize signature peptides of potential off-target protein kinases as biomarkers to determine the off-target occupancy and selectivity of the inhibitor of interest. The methods can be used in research and clinical settings for (1) minimizing the side effects of kinase-targeted therapies and (2) managing and monitoring patient toxicities.

### I. Minimizing the side-effects of a kinase-targeted therapy

Off-target toxicities may limit the use of kinase inhibitors. In one aspect, the present disclosure includes a method that includes the steps of (1) administering a kinase inhibitor under more than one condition (e.g. different levels, frequencies, and/or durations) to a test subject or subjects, (2) determining off-target kinase occupancy in a sample obtained from the test subject(s) using signature peptides using the methods described herein, and (3) optimizing dosing or regimens based on the determined levels in order to minimize off-target side effects.

### II. Managing and monitoring off-target effects

Also described herein is a method of monitoring a patient undergoing a kinase-targeted therapy, the method includes the steps of (1) determining the off-target kinase occupancy of a kinase inhibitor in a sample obtained from the patient using signature peptides after the inhibitor is administered, (2) comparing the determined level with a corresponding pre-determined level, the predetermined level being obtained from a reference sample from a reference subject in which the inhibitor caused side effect(s) or from the patient at a time point where the side effect(s) are present. The inhibitor is determined to have the undesired side effect(s) in the patient if the off-target occupancy falls above the pre-determined level.

The specific examples below are to be construed as merely illustrative, and not limitative of the remainder of the disclosure in any way whatsoever. Without further elaboration, it is believed that one skilled in the art can, based on the description herein, utilize the present disclosure to its fullest extent. All publications cited herein are hereby incorporated by reference in their entirety.

### Blood assay of kinase target engagement (%drug occupancy)

The target engagement of the BTK inhibitor ibrutinib (Impruvica) was investigated in cancer patients. Ibrutinib is a small molecule covalent irreversible BTK inhibitor targeting the Cys481 residue within the BTK ATP-binding pocket. See Fig. 2. Concentrations of ibrutinib-bound BTK were determined in patients' peripheral blood mononuclear cells (PBMCs) based on representative signature peptides derived from a chymotrypsin digestion procedure. PBMC lysates were reduced and alkylated with iodoacetamide (IAM). After alkylation, the samples were treated with chymotrypsin to generate the free (unbound) BTK peptide MANGCLL(SEQ ID NO: 1) and the inhibitor-bound BTK peptide MANGC^{[Ibrutinib]}LL (SEQ ID NO: 1).

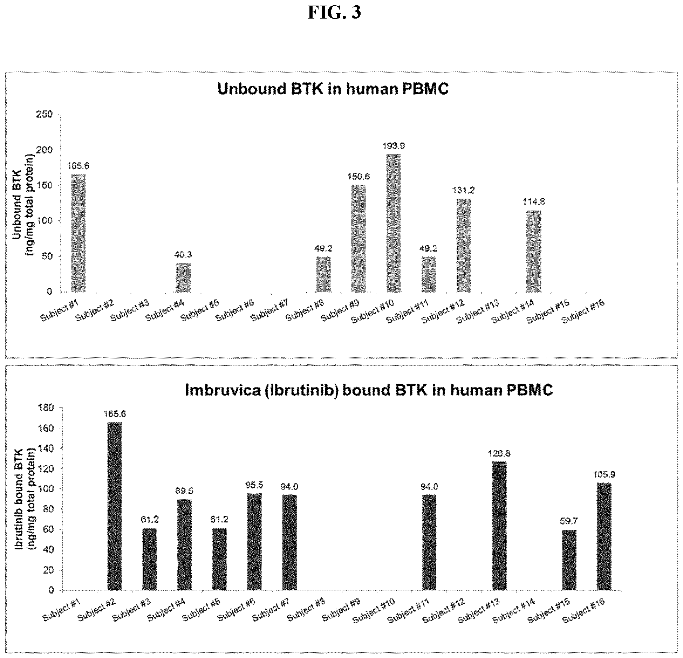

The MANGCLL (SEQ ID NO: 1) and MANGC^{[Ibrutinib]}LL (SEQ ID NO: 1) peptides were absolutely quantitated in the digested PBMC lysates using a sensitive/specific UPLC-MS/MS method. A corresponding mass-shifted, stable isotope-labeled peptide was employed as an internal standard. Injections were made onto an UPLC column using a Shimadzu SIL 30ACMP autosampler and Shimadzu LC-20AD pump. Mobile phase A was 0.1% formic acid in water. Mobile phase B was 0.1% formic acid in 90:10 acetonitrile/water (v/v). The analytes were detected with an API SCIEX TripleTOF 6600 mass spectrometer. The measured peptide concentrations were converted to protein concentrations and normalized by total protein. Data were reported in ng/ng total protein in PBMC lysate. See Fig. 3

The method would allow for the evaluation of the target engagement of ibrutinib in clinical trials and patient samples. In addition to ibrutinib, utilization of signature peptides, i.e, MANGCLL (SEQ ID NO: 1) and MANGC^{[inhibitor]}LL (SEQ ID NO: 1), is readily adaptable to any other BTK inhibitor that binds covalently at residue Cys481 (e.g., acalabrutinib and zanubrutinib). Moreover, other BTK signature peptides could be selected to investigate the target engagement of inhibitors that interact with residues other than Cys481 as well. The method can be adapted to kinase inhibitors that target any kinases, as long as the binding sites of the inhibitors within the kinases are known.

### Blood assay for overcoming genetic resistance

The method described herein can be used to develop a personalized treatment plan based on the presence or percentage of genetic mutations and/or status (e.g., quantitative levels or activation state) of the target kinase in a patient's peripheral blood cells prior to, during and after therapy. For example, the detection or increase in the percentage of the BTK C481S signature peptide MANGSLL (SEQ ID NO: 2) over the wild-type MANGCLL (SEQ ID NO: 1) peptide may alert of the patient's acquired resistance to ibrutinib, presenting the physician an opportunity to select an alternate therapy. The percentages of different BTK C481 mutations (C481S/R/P) were determined in different cancer patients. See Table 1.

**Table 1**

| | Patient #68 | Patient #81 | Patient #82 | Patient #89 |
|---|---|---|---|---|
| C481 | 21.9% | 96.5% | 81.0% | 73.7% |
| C481S | 78.1% | | | |
| C481R | | 3.5% | 19.0% | |
| C481P | | | | 26.3% |

### Assay qualification - increase in %occupancy of BTK inhibitors with treatment level

The target occupancies of small molecule covalent irreversible BTK inhibitors (ibrutinib, acalabrutinib, zanubrutinib, spebrutinib, and tirabrutinib) were investigated *in vitro.* Human whole blood was treated with increasing concentrations of the BTK inhibitors (C_{final}=0.5 to 1000 nM) in separate experiments. After incubation, a saturating amount of an ibrutinib analog, ibrutinib-d5, was spiked into each experimental sample to bind any residual (i.e., free) active kinase. Leukocytes were isolated from the sample and digested with chymotrypsin to generate the inhibitor bound BTK peptides, e.g., MANGC^{[Ibrutinib]}LL (SEQ ID NO: 1) and MANGC^{[Acalabrutinib]}LL (SEQ ID NO: 1), the ibrutinib-d5 bound peptide (MANGC^{[Ibrutinib-d5]}LL(SEQ ID NO: 1)), and the unbound non-active BTK peptide MANGCLL (SEQ ID NO: 1). The signature peptides were absolutely quantitated using the sensitive/specific UPLC-MS/MS method described above. The measured peptide concentrations were converted to protein concentrations and normalized by total protein.

For each BTK inhibitor, the percent kinase occupancy was determined as %occupancy = 100 x (bound/total), where full occupancy was confirmed at 100%. Concentrations of the active kinase in its bound and free states were determined based on the levels of inhibitor-bound signature peptides MANGC^{[Ibrutinib]}LL (SEQ ID NO: 1) and MANGC^{[Ibrutinib-d5]}LL (SEQ ID NO: 1), respectively. Concentrations of inactive kinase were based on the levels of the unbound BTK peptide MANGCLL (SEQ ID NO: 1). The total amount of BTK was given by total = active kinase (bound and unbound states) + inactive kinase.

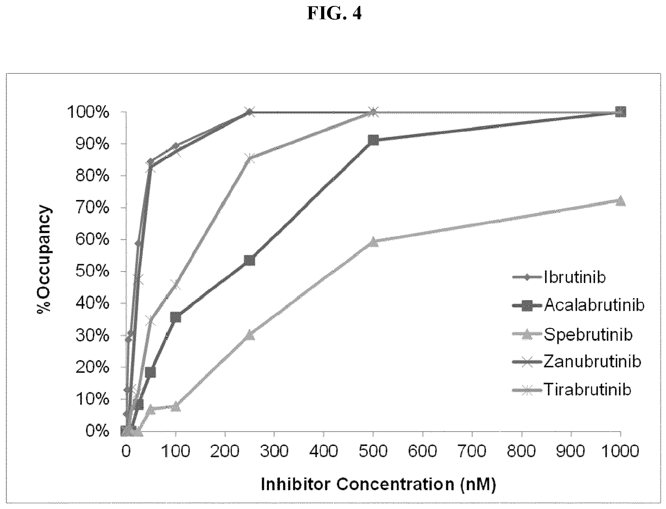

The enzyme occupancy of the BTK inhibitors are presented in Fig. 4. The BTK occupancy of each compound increased with concentration (C_{final}=0.5 to 1000 nM). The highest %occupancy was observed with ibrutinib, followed by zanubrutinib, tiribrutinib, acalabrutinib and then spebrutinib.

### Drug target engagement - %occupancy of zanubrutinib, acalabrutinib, and ibrutinib in primary samples from leukemia patients

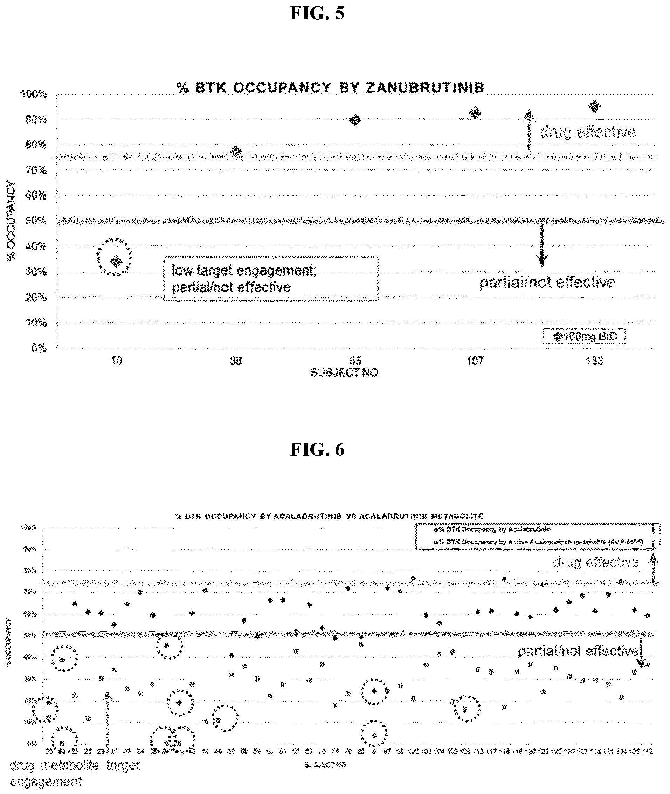

BTK %occupancy in leukemia patients on zanubrutinib, acalabrutinib, and ibrutinib therapy were determined. The results showed that one patient (#19) was not receiving full benefit from zanubrutinib therapy (i.e., %occupancy level <50%). See Fig. 5. Physicians could use this information to adjust the treatment in a rapid fashion.

The results also showed that variable metabolism accounted for different aggregate target engagement. See Fig. 6. In some patients, neither the parent drug (acalabrutinib) nor its active metabolite (ACP-5386) had achieved an efficacious level (≥50% BTK occupancy). This information would allow physicians to make real time decisions about drug choices.

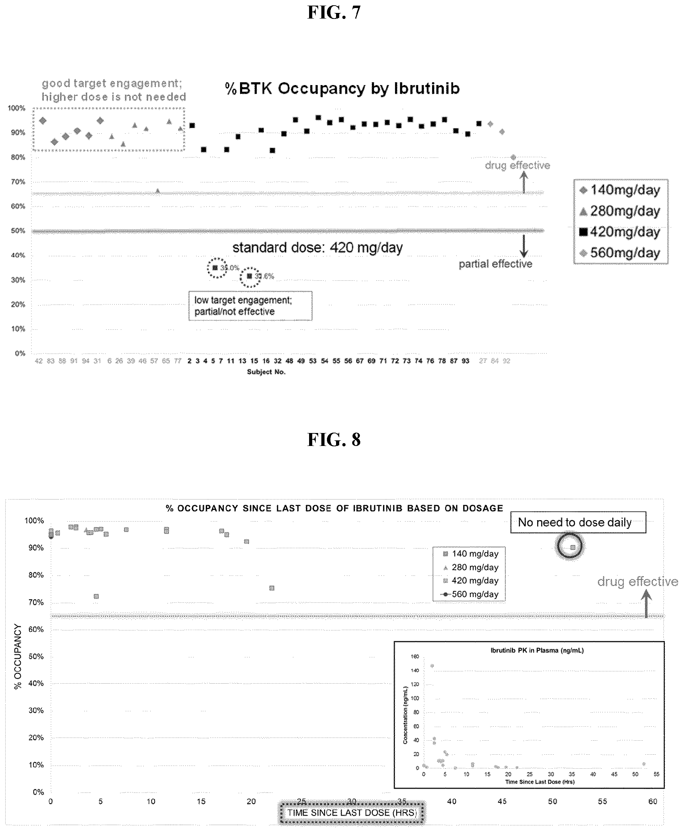

BTK %occupancy in cancer patients on different doses of ibrutinib therapy (140, 280, 420, or 560 mg/day) was determined. Although lower doses were adequate for some patients (%occupancy >50%), higher doses were not enough for some patients (#5 and 15). See Fig. 7. This information would allow physicians to manage dosing, efficacy and side effects.

By assessing drug occupancy post dose at different time points, the physician can determine whether patient requires daily or less than daily dosing, thereby minimizing side effects. See Fig. 8.

### Assessing potency on cancer and effects on normal cells.

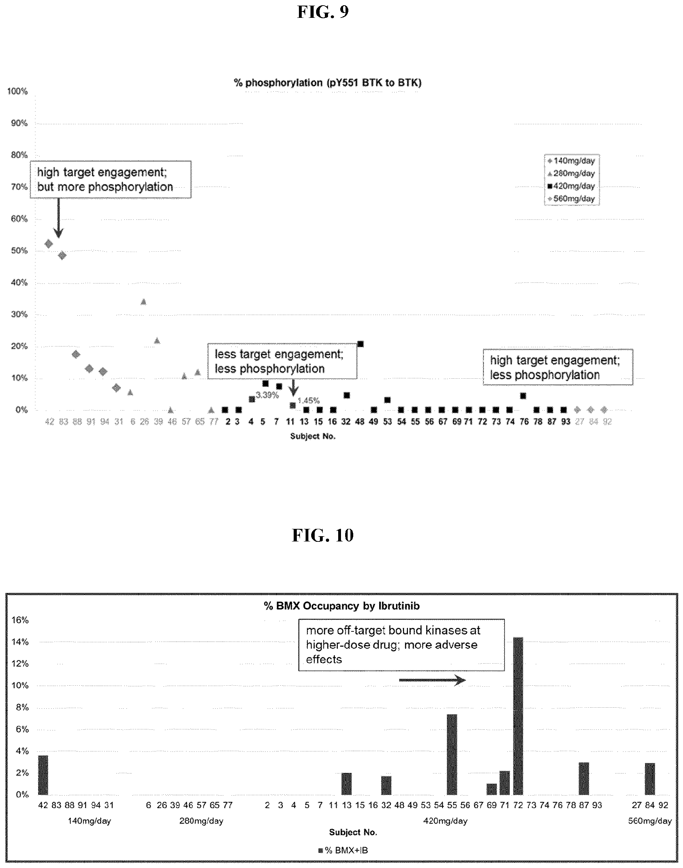

The %phosphorylation of the Tyr551 residue of BTK was measured for different cancer patients on ibrutinib therapy. See Fig. 9. Although robust target engagement was observed across all doses (i.e., 140, 280, 420, or 560 mg/day) in these patients as shown in Fig. 7, auto-phosphorylation of the Tyr551 residue (i.e., activation of BTK) might have been over-inhibited at the higher doses. Fcγ receptor (FcγR) activation of allergic reactions and immune defense mechanisms involving BTK might have been blocked. These doses could be too high. This type of data would allow physicians to assess the benefit and risk of higher doses of a drug.

### Off-target effects in leukemia patients on ibrutinib therapy

The leukocytes of leukemia patients on ibrutinib therapy were digested with chymotrypsin to generate signature peptides representative of the binding sites of a panel of protein kinases (BTK, BLK, BMX, TEC, and TXK). For each protein, levels of ibrutinib-bound and free (unbound) signature peptides (e.g., ISNGC^{Ibrutinib}LL (SEQ ID NO: 16) and ISNGCLL (SEQ ID NO: 16) for BMX) were determined by UPLC-MS/MS. The levels of signature peptides were used to determine the %occupancy of ibrutinib for each protein kinase. The BMX (off-target) %occupancy of ibrutinib in leukemia patients is shown in Fig. 10.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

## Claims

1. A method of determining target kinase occupancy of a test compound, the method comprising:
(1) obtaining a test sample or a plurality of test samples, wherein the test sample is a biological sample from a test subject that has been administered the test compound, or is a biological sample that has been contacted with the test compound;
(2) processing a first portion of the test sample to generate a population of a representative signature peptide that includes a bound signature peptide and a free signature peptide, wherein the bound signature peptide is a representative signature peptide bound to the test compound and the free signature peptide is the representative signature peptide free of the test compound, the representative signature peptide being a fragment of the target kinase that includes a binding site for the test compound;
(3) determining in the processed sample the level of the bound signature peptide and the level of the free signature peptide;
(4) obtaining the level of a bound target kinase and the level of a free target kinase in the test sample based on the level of the bound signature peptide and the level of the free signature peptide, respectively, the bound target kinase being the target kinase bound to the test compound and the free target kinase being the target kinase free of the test compound; and
(5) obtaining the target kinase occupancy of the test compound, which is the level of the bound target kinase relative to the combined level of the level of the bound target kinase and the level of the free target kinase.

2. The method of claim 1, further comprising, after step (1) but before step (2), contacting the test sample with a saturating amount of a reference compound that is capable of binding covalently and strongly to the target kinase at the binding site within the representative signature peptide, and determining in the processed sample the level of the representative signature peptide bound to the reference compound, wherein the test compound binds covalently to the target kinase.

3. The method of claim 2, further comprising:
obtaining a residual level of the target kinase in its active state but free from the test compound in the test sample based on the level of the representative signature peptide bound to the reference compound, wherein the level of the target kinase in its active state in the test sample is the combined level of the level of the bound target kinase and the residual level, and wherein the total level of the target kinase in the test sample is the combined level of the level of the target kinase in its active state and the level of the free target kinase; and
obtaining the target kinase occupancy, which is the level of the bound target kinase relative to the total level of the target kinase; optionally, the target kinase occupancy is a percent kinase occupancy calculated by 100 x (the level of the bound target kinase/the total level of the target kinase).

4. The method of claim 2, wherein the reference compound is the test compound that is stably isotope labeled or biotinylated.

5. The method of claim 1, further comprising, after step (1) but before step (2), contacting a second portion of the test sample with a saturating amount of a biotinylated probe that is capable of binding irreversibly to a site in the target kinase that includes the binding site of the test compound, processing the second portion of the test sample to generate a probe-bound signature peptide, which is the representative signature peptide bound to the biotinylated probe, and determining the level of the probe-bound signature peptide, wherein the test compound binds reversibly or non-covalently to the target kinase.

6. The method of claim 5, further comprising, dissociating the test compound from the target kinase before contacting the second portion with the biotinylated probe; and/or
obtaining the level of the target kinase in its active state based on the level of the probe-bound signature peptide, wherein the total level of the target kinase in the test sample is the combined level of the level of the target kinase in its active state and the level of the free target kinase; and
obtaining the target kinase occupancy, which is the level of bound target kinase relative to the total level of the target kinase; optionally the target kinase occupancy is a percent kinase occupancy calculated by 100 x (the level of bound target kinase/the total level of the target kinase).

7. The method of claim 1, further comprising:
after step (1), contacting a third portion of the test sample with a saturating amount of a reference compound that is capable of binding irreversibly to a site in the target kinase that includes the binding site of the test compound, and processing the third portion of the test sample to remove any protein not bound to the reference compound and excess reference compound;
dissociating the test compound from any bound protein, precipitating the dissociated protein, and measuring the levels of the test compound and the reference compound in the supernatant, wherein the level of total protein bound to the test compound is given by the level of the test compound in the supernatant, and the amount of total protein not bound to the protein (unbound) is given by the level of the reference compound in the supernatant, and the amount of total kinase equals the amount of the bound kinase plus the unbound kinase; and
obtaining a total target occupancy, which is the level of bound total kinase relative to the level of total kinase; optionally, the total target occupancy is a percent occupancy calculated by 100 x (the level of bound total kinase/the level of total kinase).

8. The method of claim 7, further comprising:
obtaining an off-target occupancy of the test compound by subtracting the target kinase occupancy from the total target occupancy, wherein the off-target occupancy is the occupancy of the test compound to one or more unintended targets of the test compound; and/or
after step (1), fractionating out from a portion of the test sample a test compound-kinase complex by adding ammonium sulfate to precipitate out other proteins, recovering the supernatant, adding more ammonium sulfate to precipitate out the test compound-kinase protein complex, and collect the precipitated complex for analysis.

9. The method of claim 1:
wherein the population of representative signature peptide is generated by a specific proteolytic cleavage procedure, by a non-specific proteolytic procedure, or a chemical-based procedure; and/or
wherein the determining step is conducted using a ligand-binding assay, liquid chromatographic (LC), capillary electrophoretic (CE) based technique, or a combined LC or CE mass spectrometric (MS) procedure; and/or
wherein the target kinase is or is not the intended target of the compound.

10. The method of any preceding claim, wherein the representative signature peptide contains MANGCLL (SEQ ID NO: 1), MANGSLL (SEQ ID NO: 2), MANGYLL (SEQ ID NO: 4), MANGRLL (SEQ ID NO: 5), MANGALL (SEQ ID NO: 6), MANGFLL (SEQ ID NO: 7), MANGTLL (SEQ ID NO: 8), MANGPLL (SEQ ID NO: 9), ISNGCLL (SEQ ID NO: 16), EFMEHGCLSDY (SEQ ID NO: 17), LPSGCL (SEQ ID NO: 18), LPSGCLRDF (SEQ ID NO: 19), MERGCLL (SEQ ID NO: 20), MENGCLL (SEQ ID NO: 21), or MPHGCLL (SEQ ID NO: 22).

11. A method of evaluating the likelihood of a subject responding to a candidate compound, the method comprising:
(1) obtaining a test sample, wherein the test sample is a biological sample from the subject;
(2) processing the test sample to generate a population of representative signature peptide that includes a modified signature peptide and a wild-type signature peptide, the representative signature peptide being a fragment of a kinase that is a target of the candidate compound, wherein the representative signature peptide includes a site that, if mutated or post-translationally modified, can affect responsiveness to the candidate compound, the modified signature peptide being the mutated or modified representative signature peptide, and the wild-type signature peptide being the representative signature peptide free from the mutation or modification;
(3) determining in the processed sample the level of the modified signature peptide and the level of the wild-type signature peptide;
(4) obtaining the presence or percentage of the mutation or modification based on the level of the modified signature peptide and the level of the wild-type signature peptide; and
(5) evaluating the subject's likelihood of responding to the candidate compound based on the presence or percentage, wherein a presence or high percentage indicates a low likelihood.

12. The method of claim 11, wherein:
the mutation or modification is a BTK genetic mutation, optionally, the mutation or modification is selected from C481S, C481F, C481Y, C481R, C481A, C481T, T474I, T474M, T474S, T474P, T316A, and L528W, or an auto-phosphorylation event selected from pY223, pY551, pY225, pY344, and pY361.

13. A method of evaluating the likelihood of a subject responding to a candidate compound, the method comprising:
(1) obtaining a test sample, wherein the test sample is a biological sample from the subject;
(2) processing the test sample to generate a population of representative signature peptide that includes a phosphorylated signature peptide and a non-phosphorylated signature peptide, the representative signature peptide being (i) a fragment of a kinase that includes a portion of the activation segment containing the phosphorylatable residue that is phosphorylated upon activation of the kinase, or (ii) a fragment of a substrate including a phosphorylatable residue of the kinase, the phosphorylated signature peptide being the representative signature peptide phosphorylated at the phosphorylatable residue and the non-phosphorylated signature peptide is the representative signature peptide lacking the phosphorylation, wherein the kinase is a target of the candidate compound;
(3) determining in the processed sample the level of the phosphorylated signature peptide and the level of the non-phosphorylated signature peptide;
(4) obtaining the level of activated kinase or the level of phosphorylated substrate of the kinase based on the level of the phosphorylated signature peptide and the level of the non-phosphorylated signature peptide; and
(5) evaluating the subject's likelihood of responding to the candidate compound based on the level or percentage of the activated kinase or the phosphorylated substrate of the kinase, wherein a higher level or percentage indicates that the subject has a likelihood of being resistant to the candidate compound.

14. The method of claim 1, 9 or 11, wherein the candidate compound is an inhibitor of a kinase selected from protein kinase B (PKB), tyrosine-protein kinase BLK, tyrosine-protein kinase BMX, Bruton's tyrosine kinase (BTK), epidermal growth factor receptor (EGFR), receptor tyrosine-protein kinase erbB-2 (ERBB2), receptor tyrosine-protein kinase erbB-4 (ERBB4), extracellular signal-regulated kinase (ERK), tyrosine-protein kinase Fgr (FGR), Fyn-related kinase (FRK), tyrosine-protein kinase Fyn (FYN), tyrosine-protein kinase HCK, tyrosine-protein kinase ITK/TSK/LYK, Janus kinase 3 (JAK3), lymphocyte-specific protein tyrosine kinase (LCK), tyrosine-protein kinase Lyn (LYN), ribosomal protein S6 kinase A3 (RPS6KA3), tyrosine-protein kinase Src (SRC), spleen tyrosine kinase (SYK), tyrosine-protein kinase Tec (TEC), tyrosine-protein kinase TXK, tyrosine-protein kinase Yes, and tyrosine-protein kinase ZAP-70.

15. The method of claim 12 or 14, wherein the determining step is conducted using a ligand binding assay, liquid chromatographic (LC) based technique, capillary electrophoretic (CE) based technique, or a combined LC or CE mass spectrometric (MS) procedure.
